# EUROPÄISCHE PATENTSCHRIFT

(11) **EP 1 225 925 B1**
(45) Veröffentlichungstag und Bekanntmachung des Hinweises auf die Patenterteilung: **05.11.2003**
(21) Anmeldenummer: 00965752.9
(22) Anmeldetag: 14.08.2000
(51) Int. Cl.: A61L 9/00

(54) **VORRICHTUNG UND VERFAHREN ZUM GENERIEREN UND ABFÜLLEN VON DUFTKOMPOSITIONEN**
DEVICE AND METHOD FOR GENERATING AND DISPENSING AROMATIC COMPOSITIONS
DISPOSITIF ET PROCEDE POUR GENERER ET DISPENSER DES PARFUMS

(30) Priorität: 13.08.1999 DE 19938405
(43) Veröffentlichungstag der Anmeldung: 31.07.2002
(73) Patentinhaber: Bork, Karl-Heinz, 37627 Deensen (DE)
(72) Erfinder: RUETZ, Stefan, 80807 München (DE); BORK, Karl, Heinz, 37627 Deensen (DE); BREU, Christian, D-85748 Garching (DE)
(74) Vertreter: Zipse + Habersack
(86) Internationale Anmeldenummer: DE0002742
(87) Internationale Veröffentlichungsnummer: WO01012239

(56) Entgegenhaltungen:
- WO-A-99/16476
- US-A- 5 591 409
- US-A- 5 724 256
- US-A- 6 024 783

## Beschreibung

Die vorliegende Erfindung betrifft eine Vorrichtung und ein Verfahren zum Aufbringen/Austragen von Duftsubstanzen in die Luft oder auf einen Duftträger, z.B. ein Papierblatt oder Papierstreifen oder auf die Haut, die insbesondere zur Erzeugung von Duftkompositionen geeignet sind, nach den Ansprüchen 1 und 8.

Parfums werden bislang in Drogerien und Parfümerien von Hand auf einen Duftträger gesprüht, der in der Regel als Papierstreifen ausgebildet ist. Aufgrund des manuellen Auftrags schwankt die aufgebrachte Parfümmenge erheblich und regelmäßig ist Verkaufspersonal notwendig, um das Parfum aufzutragen. Es ist daher Aufgabe der Erfindung, eine Vorrichtung und ein Verfahren anzugeben, das einen reproduzierbaren und gleichmäßigen Auftrag/Austrag von Duftsubstanzen auf einen Duftträger bzw. in die Luft bei sparsamer Verwendung der Duftsubstanz ermöglicht. Diese Aufgabe wird durch eine Vorrichtung gemäß Anspruch 1 und ein Verfahren gemäß Anspruch 28 gelöst. Vorteilhafte Weiterbildungen der Erfindung sind Gegenstand der entsprechenden ünteransprüche.

Die Dokumente US-A-5 724 256 und US-A-5 591 409 beschreiben eine Vorrichtung zum Genevienen von Duftkompositionen die einen Behälter, eine Austragsvorrichtung und einen Rechner aufweist.

Die erfindungsgemäße Vorrichtung ist zum Aufbringen wenigstens einer Duftsubstanz auf einen Duftträger konzipiert und hat wenigstens mehrere Behälter zur Aufnahme mindestens einer Duftsubstanz. Dies kann ein Einzelstoff, ein Duftbaustein oder bereits eine Duftmischung sein. Zum Überführen einer Kleinstmenge der Duftsubstanz aus dem Behälter auf den Duftträger ist wenigstens eine Auftragsvorrichtung vorgesehen. Die kann ein Druckkopf eines herkömmlichen Druckers, z.B. Tintenstrahldruckers sein. Es sind jedoch alle Vorrichtungen z.B. auf piezoelektrischer oder elektromagnetischer Basis denkbar, die in der Lage sind, Kleinstmengen im µl-Bereich bis unterhalb des pl (Pikoliter)-Bereichs zu fördern. Die Duftsubstanz wird dann durch die Auftragsvorrichtung als Mikrotropfen oder Nebel aufgebracht. Die Auftragsvorrichtung wird von mindestens einer Steuerung betätigt, die mittels eines Rechners Aktivierungsdaten für die Auftragsvorrichtung aus den Mengen/Mischungsdaten errechnet, die eine Person über wenigstens einer Eingabeeinrichtung eingegeben hat. Vorzugsweise in der Steuerung ist ein Rechner zum Generieren von Aktivierungs- und/oder Dosierungssignalen aus den Aktivierungsdaten vorgesehen.

Die Vorrichtung kann somit dazu dienen, auf einen Duftstreifen eine Duftsubstanz auf ein Duftfeld aufzubringen. Sie kann auch dazu verwendet werden, auf ein Blatt Papier , z.B. einen Brief einen Duft aufzubringen, wobei die Aufbringung vorzugsweise zusammen mit dem Aufdrucken des Textes erfolgen kann. Die Duftsubstanz könnte in diesem Fall sogar mit einer Tinte gemischt in einem Tintenbehälter vorgesehen sein, so daß beim Druckvorgang neben der Tinte auch die Duftsubstanz auf ein Blatt Papier aufgebracht wird. Der Tintenbehälter kann in diesem Fall aus einem eng vernetzten Polymer bestehen, um ein Entweichen der flüchtigeren Bestandteile der Duftsubstanz zu unterbinden.

Wenn die Vorrichtung mehrere Behälter zur Aufnahme unterschiedlicher Duftsubstanzen aufweist, wenigstens eine Eingabeeinrichtung für die Eingabe von Mengen/Mischungsdaten einer Duftkomposition an die Steuerung, und wenigstens einen, vorzugsweise in der Steuerung vorgesehenen, Rechner zum Generieren von Akitivierungs- bzw. Dosierungssignalen für die Auftragsvorrichtung aus den Mengen/Mischungsdaten, können mit einer derartigen Vorrichtung auch Duftkompositionen erzeugt werden.

Denn Düfte wie zum Beispiel Eau de Toilette, After Shaves und Parfums werden bislang von Kosmetikfirmen komponiert. Die Duftkompcsition wird von professionellen Parfümeuren durchgeführt, die eine große Erfahrung haben, wie verschiedene Düfte zusammenwirken und die die Qualität der komponierten Düfte gut beurteilen können. Ein nicht unbeachtlicher Teil der Verbraucher findet dennoch in den am Markt angebotenen Düften nicht den individuell passenden Duft. Für derartige Kunden wurden bislang Sets von Parfümgrundsubstanzen angeboten, die die Verbraucher nach eigenem Gutdünken mischen konnten. Das Ergebnis dieser in gewisser Weise nicht fachmännischen Duftkompositionen war jedoch teilweise verheerend, da nicht alle Duftsubstanzen in beliebiger Weise miteinander gemischt werden können. Dies ist jedoch eine Kenntnis bzw. eine Erfahrung, die dem Endverbraucher als Laien fehlt.

Die Vorrichtung erlaubt daher dem Endverbraucher eine individuelle Anpassung einer Duftkomposition an seine individuellen Bedürfnisse unter Beibehaltung einer hohen Qualität. Dem professionellen Parfümeur wird zudem ein Mittel bereitgestellt, das es ihm ermöglicht, mit geringem Aufwand neue Duftkompositionen zu testen.

So weist die Vorrichtung mehrere Behälter zur Aufnahme unterschiedlicher Duftsubstanzen auf. Weiterhin ist wenigstens eine Auftragsvorrichtung zur separaten Überführung einer Kleinstmenge einer Duftsubstanz auf einen Duftträger, z.B. auf ein aufgerauhtes Papier und mindestens eine Steuerung für die Auftragsvorrichtung vorgesehen. Die Auftragsvorrichtung ist hierbei vorzugsweise derart ausgebildet, daß mehrere Duftsubstanzen gleichzeitig oder sukzessive entweder überdekkend oder eng nebeneinander auf den Duftträger aufgebracht werden. Auf diese Weise wird auf den Duftträger entsprechend der Anzahl und dem Mengenverhältnis der aufgebrauchten Duftsubstanzen eine Duftkomposition homogen aufgebracht, die dem Duft eines fertig gemischten Duftes entspricht.

Die Vorrichtung zum Aufbringen und zur Erzeugung von Duftkompositionen hat eine Eingabeeinrichtung, durch welche eine Bedienperson, z.B. ein Endverbraucher oder ein Parfümeur die gewünschte Mischung eingibt bzw. einstellt. Diese Eingabeeinrichtung kann durch eine Tastatur, durch einen Touch Screen oder durch einen Joystick in Verbindung mit einem Bildschirm gebildet sein. Eine weitere Möglichkeit besteht darin, die Eingabeeinrichtung zusammen mit der Steuerung der Auftragsvorrichtung in einem PC vorzusehen. Deshalb ist im Bereich der Eingabeeinrichtung vorzugsweise auch eine Anzeige vorgesehen, die die Position der gewählten Duftmischung in einer genealogischen Tafel angibt. In dieser genealogischen Tafel ist angegeben, welche Duftrichtung die gewählte Duftkomposition aufweist, z.B. fruchtig, frisch, blumig, süß, orientalisch, tabakartig, vanillisch, animalisch. Die Steuerung kann hier auch einen Speicher für eine Mischungsmatrix aufweisen, der eine unbeschränkte Mischung der vorhandenen Duftsubstanzen unterbindet, um eine gewisse Qualität und Homogenität der selbst zusammengestellten Duftmischungen zu gewährleisten. Diese Qualität kann jedoch auch schon bereits dadurch gewährleistet werden, daß die in den Behältern bereitgestellten Substanzen vorgemischte Duftsubstanzen sind, die bei gegenseitiger Mischung ihre Qualität nicht verlieren. Der Qualitätsanspruch wird hier also bereits durch eine Vorauswahl der in den Behältern bereitgestellten Duftsubstanzen gewährleistet.

Prinzipiell ist es möglich, eine Auftragsvorrichtung, z.B. eine kleine elektrische oder piezoelektrische Druckvorrichtung mit einer Düse zum Auftragen der Substanzen aus allen Behältern zu verwenden. In dem Fall müßten jedoch die unterschiedlichen Behälter sukzessive mit der Vorrichtung verbunden werden. Die Druckvorrichtung müßte gegebenenfalls vor dem Verbinden mit einem neuen Behälter gereinigt werden. Deshalb ist es vorteilhaft, wenn für jeden Behälter eine eigene Auftragsvorrichtung, z.B. eine eigene Tintenstrahlspritzdüse vorgesehen ist, in welchem Fall die unterschiedlichen Duftsubstanzen gleichzeitig auf den Duftträger aufgebracht werden können. Das ist auch deswegen von Vorteil, da bei einer sukzessiven Aufbringung der Duftsubstanzen der Gesamteindruck der Duftkomposition aufgrund der zeitlichen Einflüsse verfälscht wird.

Als Auftragsvorrichtung kann beispielsweise ein herkömmlicher Druckkopf verwendet werden, wie er in Tintenstrahldruckern, Bubble-Jet-Druckern und Thermodruckern verwendet wird. Der Auftrag erfolgt hier durch Spritzen eines Mikrostrahles oder - Nebels auf den Druckträger oder durch Verdampfen eines Mikrotröpfchens. Die den unterschiedlichen Behältern zugeordneten Düsen des Druckkopfes sind hierbei vorzugsweise alternierend angeordnet, so daß eine homogene Verteilung der Duftpunkte auf dem Duftträger erzielt wird. Hierdurch wird eine gleichmäßige homogene Mischwirkung erzielt, was zur Folge hat, daß der Duft an jeder Stelle des Duftträgers identisch zusammengesetzt ist. Selbstverständlich können zum Aufbringen der Duftsubstanzen auf den Duftträger auch andere Auftragsvorrichtungen wie zum Beispiel Mikropumpen, Ultraschallzerstäuber etc. verwendet werden.

Wenn von Kleinstmengen die Rede ist, handelt es sich um Mengen, die ausreichend sind, um auf einem Duftträger, z.B. einem Papierstreifen eine ausreichende Duftwirkung bereitzustellen. Die hierfür erforderlichen Mengen an Duftsubstanz liegen in der Regel unterhalb des Bereichs von Mikrolitern bis in den Pikoliterbereich.

Wenn die Duftsubstanzen durch einen Druckkopf aufgetragen werden, kann zusätzlich ein Druckkopf für den Auftrag von Tinte vorgesehen sein, durch welchen sich im Bereich des Düftträgers oder in einem Beschriftungsbereich neben dem Duftträger Beschriftungen aufbringen lassen. Derartige Beschriftungen können z.B. den Namen der Duftkreation enthalten, eine Mischungsformel, die z.B. zur Weitergabe an eine Abfüllstation auch in maschinenlesbarer Schrift vorgesehen sein kann (Barstrichcode) und das Datum der Duftzusammenstellung. Selbstverständlich können auch weitere Angaben wie z.B. die Art des Duftes auf einem Beschriftungsfeld angegeben werden. Der Druckkopf für die Beschriftung kann auch mit dem Druckkopf für den Auftrag der Duftsubstanzen integriert sein, so daß der Druckkopf sowohl mit Behältern für Duftsubstanzen als auch mit wenigstens einem Tintenbehälter verbunden ist. Eine derartige Vorrichtung ließe sich in einfacher Weise in einer Art adaptiertem Tintenstrahl-, Bubble-Jet oder Thermodrucker realisieren.

Der Drucker könnte einen Einschub für die Eingabe eines Papierstreifens haben, der dann bedruckt wird oder er könnte als herkömmlicher DIN-A-3- oder DIN-A-4-Drucker ausgebildet sein, in den horizontal perforierte DIN-A-4-Blätter eingelegt werden. Nach Aufdrucken der Duftkomposition und der Beschriftung wird der Streifen dann so weit vorgeschoben, daß er an der Perforation manuell oder automatisch abgetrennt werden kann. Im Falle einer automatischen Trennung braucht nicht einmal eine Perforation vorgesehen werden.

Falls die generierte Duftkomposition als vorteilhaft erachtet wird, kann in dem Drucker auch eine Beschriftungsposition oder ein Beschriftungsbereich für ein Etikett oder für das Parfumflakon selbst vorgesehen sein. Das Etikett bzw. der Flakon wird dann direkt mit dem Namen und eventuell einem maschinenlesbaren Mischungscode bedruckt. Das Etikett bzw. der Flakon kann dann in eine Abfüllstation eingestellt werden, die das Etikett bzw. den Flakon auf die maschinenlesbare Mischungsformel abscannt und somit das Mischungsverhältnis der Duftsubstanzen erhält. Oder die Abfüllstation erhält die Mischungsdaten direkt von der Duftgenerierungsvorrichtung zum Beispiel über eine Leitung oder per Funk. Auf diese Weise wird sichergestellt, daß die in der Duftgenerierungsvorrichtung erzeugte Duftkomposition auch tatsächlich der Mischung entspricht, die nachher in den Parfümflakon abgefüllt wird. Eine Verwechslung der Behälter etc. kann aufgrund der automatischen Steuerung ausgeschlossen werden. Durch die vorrichtung nach Anspruch 1 lassen sich somit Düfte nicht nur generieren, sondern gleich in kleinen Mengen, d.h. Mengen von 1 bis 500 ml abfüllen. Die Abfüllstation weist hierfür mehrere Behälter mit unterschiedlichen Duftsubstanzen auf, deren Größe aber deutlich über der Behältergröße der Duftgenerierungsvorrichtung liegt. Jeder Behälter ist mit einer Mikroförderpumpe versehen, die durch eine zentrale Steuerung angesteuert werden. Die Mischungs- und Mengendaten erhält die Abfüllstation durch die Duftgenerierungsvorrichtung und gegebenenfalls durch die manuell eingegebene oder automatisch festgestellte Größe des Parfümflakons.

Die Mikroförderpumpen der Abfüllstation münden in einen Befüllungsbereich für einen kleinen Behälter. Der kleine Behälter kann entweder ein Zwischenbehälter sein, der zum manuellen oder automatischen Mischen der zusammengestellten Komposition verwendet wird oder gleich der Parfümflakon, der ebenfalls noch einmal manuell oder automatisch gerührt bzw. geschüttelt werden kann, um die erzeugte Duftkomposition zu homogenisieren.

Die Anzahl der in der Duftgenerierungsvorrichtung bzw. in der Abfüllstation vorgesehenen Behälter liegt vorzugsweise zwischen 5 und 100. Eine größere Anzahl an Behältern für Duftsubstanzen ermöglicht eine differenziertere Bildung von Düften in unterschiedlichen Duftrichtungen.

Wenn man die Drucktechnologie herkömmlicher Tintenstrahldrucker verwendet, die eine Auflösung von 720 dpi haben, erhielte man bei 100 verschiedenen Behältern für Duftsubstanzen und alternierender Anordnung der in den verschiedenen Duftsubstanzen zugeordneten Düsen einen maximalen Abstand zweier Duftsubstanzen von 1,7 mm. Der regelmäßige Abstand zweier Duftpunkte voneinander würde in dem Fall 3,4 µm betragen. Dies zeigt, daß sich durch die Verwendung herkömmlicher Tintendrucksysteme eine absolut homogene Verteilung der unterschiedlichen Duftsubstanzen auf einem Duftfeld bewirken läßt. Das Duftfeld kann beispielsweise eine Fläche von 2 cm x 3 cm haben. Neben dem Duftfeld ist dann vorzugsweise ein über die gesamte Breite eines DIN-A-4-Blattes sich erstreckender Beschriftungsbereich vorgesehen, in welchem der Duftname, die Herstellerfirma der Duftsubstanzen als auch die Formeln und evtl. ein maschinenlesbarer Code für die Duftmischung angegeben sein kann. Vorzugsweise können auch weitere Daten wie zum Beispiel das Datum oder der Name des Einzelhandelsgeschäftes bzw. eines Verkäufers auf dem Beschriftungsfeld angegeben sein.

Als Duftträger wird vorzugsweise herkömmliches oder spezielles Papier verwendet, welches eine gute Aufnahmefähigkeit für die Duftsubstanzen aufweist und die Duftsubstanzen in ihrer Wirkung nicht beeinflußt. Derartige Papiere werden bereits in Parfümerien verwendet und sind an sich bekannt.

Eine andere Anwendung der Erfindung ist ein Duftcomputer, d.h. ein kleines tragbares Gerät, das über eine Eingabeeinrichtung, z.B. in der Form eines Touchscreen und Eingabetasten verfügt, um eine Duftsubstanz oder Duftmischungen auszuwählen. Das Gerät enthält in der Steuerung oder Eingabevorrichtung vorzugsweise eine Mischungsmatrix, die bestimmte Mischungen von Duftsubstanzen erlaubt und andere unterbindet, um so ein gewisses Niveau der ausgewählten Duftzusammensetzungen zu gewährleisten. Die Steuerung errechnet aus den eingegebenen Menge- und Mischungsdaten Aktivierungsdaten für wenigstens eine Zerstäuberdüse oder eine andere Zerstäubervorrichtung, wodurch ein ausgewählter Duft/Duftkomposition in die Luft gestäubt wird. Hier kann entweder für jede Duftsubstanz, bzw. jeden Behälter für eine Duftsubstanz eine eigene Zerstäubervorrichtung vorgesehen sein oder die zu mischenden Duftsubstanzen werden in eine Mischkammer übertragen, von wo sie nach einem Mischvorgang mittels der Zerstäubervorrichtung vorgemischt in die Luft ausgetragen werden. Selbstverständlich können auch unterschiedliche Kartuscheneinsätze mit unterschiedlichen Duftsubstanzen für den Duftcomputer vorgesehen werden. Diese Kartuschen werden dann durch die Steuerung identifiziert und in der Eingabeeinrichtung können über eine Anzeige andere Kartuscheneinsätze oder der Wechsel von Kartuscheneinsätzen mit Duftsubstanzen angefordert werden.

Die ERfindung wird nachfolgend anhand von Ausführungsbeispielen in der schematischen Zeichnung beschrieben. In dieser zeigen:
- Fig. 1: einen Papierstreifen als Duftträger,
- Fig. 2: ein schematische Blockdarstellung einer Vorrichtung zum Austesten und Abfüllen einer Duftkomposition, und

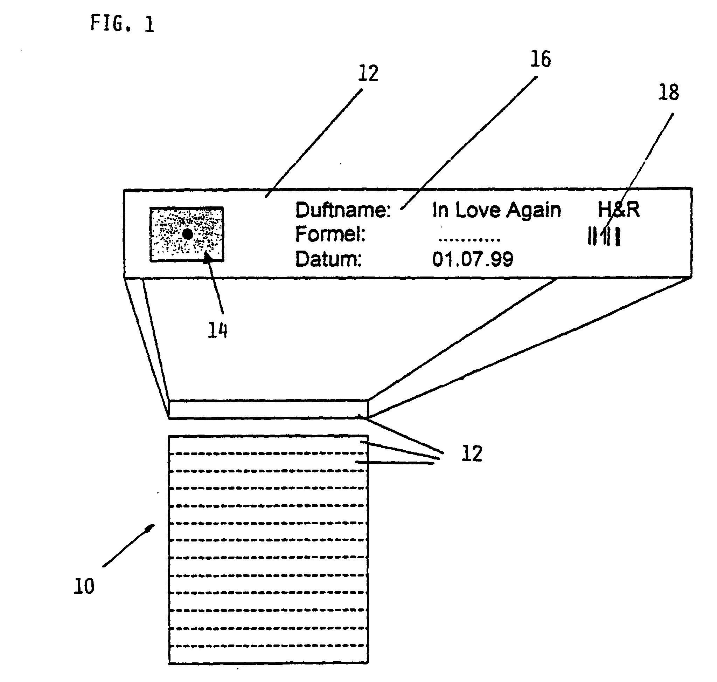

Die beiliegende Zeichnung zeigt in Fig. 1 einen Duftträger 10 aus Papier im DIN-A-4-Format zum Bedrucken in einer erfindungsgemäßen Duftgenerierungsvorrichtung.

Der Duftträger 10 wird aus einem DIN-A-4-Blatt eines speziell aufnahmefähigen Duftträgerpapiers, das in horizontale Abschnitte 12 unterteilbar ist, hergestellt. Die Unterteilung kann z.B. durch Perforationen zwischen den Abschnitten 12 gebildet sein. In der Druckvorrichtung wird über eine Eingabeeinrichtung eine gewünschte Mischung an Duftsubstanzen eingestellt, die dann auf ein Duftfeld 14 des Duftstreifens 12 aufgebracht wird. Der Duftstreifen 12 enthält neben dem Duftfeld 14 ein Beschriftungsfeld 16, in welchem unterschiedliche Informationen zur Duftkomposition angegeben sein können. Der Duftstreifen 12 mit Duftfeld 14 und Beschriftungsfeld 16 kann nach dem Bedrucken entweder manuell oder automatisch, z.B. an Perforationslinien getrennt werden. Das Beschriftungsfeld 16 enthält vorzugsweise auch einen Barcode 18, der Informationen über die Duftsubstanzen und deren Mischungsverhältnis enthält. Dieser Barcode kann z.B. danach in einer Abfüllstation gelesen und dort zum automatischen Abfüllen der richtigen Duftkomposition verwendet werden.

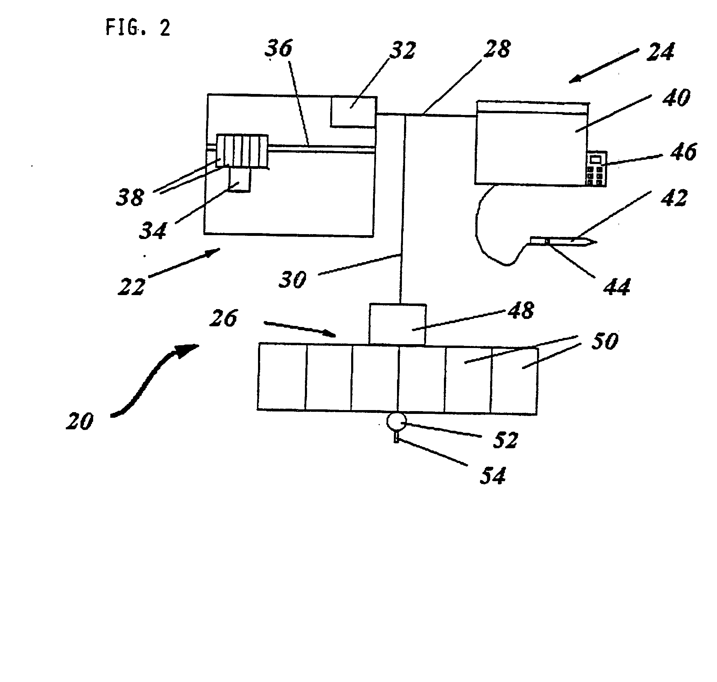

Fig. 2 zeigt eine Vorrichtung 20 mit einer Druckstation 22, einer Eingabeeinrichtung 24 und einer Abfüllstation 26, die über einen Datenbus 28,30 miteinander verbunden sind.

Die Druckstation ist in Form eines Tintenstrahldruckers ausgebildet und hat eine Steuerung 32, die einen Druckkopf 34 und einen nicht dargestellten Blattvorschub und Betätigungsmechanismus steuert, mittels dessen der Druckkopf 34 an einer Horizontalführung 36 in bekannter Weise verschiebbar gehalten ist. Mit dem Druckkopf 34 sind mehrere (sechs) Behälter 38 verbunden, in denen Duftsubstanzen und/oder Tinte und/oder Gemische von beiden enthalten sind. Diese Behälter sind in Kartuschenform auswechselbar gehalten, wie es von der Technologie bei Tintenstrahldruckern hinlänglich bekannt ist.

Die Eingabeeinrichtung 24 ist als Touchscreen 40 ausgebildet, der eine genealogische Karte aufweist. Aus dieser genealogischen Karte sind Duftrichtungen, Basisdüfte als auch bekannte Duftkompositionen verzeichnet. Mit einem Griffel 42 wird der Touchscreen 40 an der gewünschten Stelle berührt und damit der gewünschte Duft ausgesucht. Mittels einer Druckknopfes 44 wird dann der Druckvorgang initiiert. Der Druckvorgang kann auch über ein Steuerfeld 46 initiiert werden, das neben dem Touchscreen 40 angeordnet ist und zur Abgabe von Signalen an die Druckvorrichtung 22 und die Abfüllstation 26 vorgesehen ist. Das Steuerfeld 46 kann jedoch auch in dem Touchscreen 40 vorgesehen sein. Die Daten werden dann über den Bus 28 an die Steuerung 32 der Druckvorrichtung 22 gesandt, die mittels eines Rechners aus den Daten der Eingabeeinrichtung 24 Aktivierungssignale für die Betätigung und Bewegung des Druckkopfes 34 generiert. Der gewünschte ausgesuchte Duft wird dann beispielsweise auf einen Duftstreifen gedruckt.

Soll eine kleine Menge im Bereich von 5 bis z.B. 500 ml in eine Parfümflakon abgefüllt werden, so wird ein entsprechender Befehl in das Steuerfeld 46 eingegeben, worauf Daten über den Bus 30 an die Steuerung 48 der Abfüllstation 26 gesandt werden.

Die Abfüllstation 26 verfügt neben der Steuerung 48 über mehrere Großbehälter 50, die größere Mengen an Duftsubstanzen, z.B. von 100 ml bis in den Literbereich enthalten. Unter den Großbehältern 50 sind eine Reihe von Mikrodosierpumpen angeordnet, die jeweils mit einem Großbehälter verbunden sind. Die Ausgänge der Mikrodosierpumpen 50 münden in ein Abgaberohr 54, unter das ein Parfümflakon gestellt werden kann.

Die Mikrodosierpumpen 52 werden von der Steuerung 48 derart angesteuert, daß die abgefüllte Duftzusammensetzung genau der in der Druckvorrichtung 22 aufgedruckten Duftzusammensetzung entspricht.

Eine Duftkomposition wird in der Regel wie folgt ausgewählt und hergestellt.
An der Eingabeeinrichtung 24 wählt man sich zuerst Basissubstanzen aus, welche aus Einzelstoffen, Duftbausteinen oder bereits vorgemischten Mischungen bestehen können. Die interessierenden Basissubstanzen werden zuerst einzeln in der Druckvorrichtung 22 auf einen Duftstreifen 12 gedruckt. Der Benutzer hat so die Möglichkeit, die Art und Wirkung der Basisdüfte für seine Duftkomposition separat zu beurteilen. Er kann somit die interessierenden Substanzen einzeln riechen. Aus dieser Beurteilung stellt er nun seine Komposition durch Auswahl auf dem Touchscreen 40 und dem Steuerfeld 46 zusammen. Diese Duftkomposition wird nun wiederum in der Druckvorrichtung 22 auf einen Streifen 12 gedruckt. Hierbei werden die Düsen des Tintenstrahldruckkopfes durch die Steuerung 32 derart angesteuert, dass homogen über die Fläche verteilt eine Mischung von Duftsubstanzen aufgebracht wird, die der gewählten Zusammensetzung entspricht. Eine Dosierung kann dadurch erfolgen, dass z.B. eine Düse für die Duftsubstanz a dreimal betätigt wird, während die Düse für die Duftsubstanz b nur einmal betätigt wird. Auf diese Weise lassen sich beliebige Mischungen erzeugen.

Stimmt der Benutzer seiner Komposition zu, kann entweder gleich die Abfüllstation 26 über den Bus 28 und 30 betätigt werden, wodurch dort die gewünschte Zusammensetzung von Duftsubstanzen aus den Großbehältern 50 in ein Parfümflakon abgefüllt wird. Die Komposition kann zudem in einem Speicher des Systems und/oder einem Datenträger, z.B. auf dem Duftstreifen als Barcode, gespeichert werden, wodurch der Benutzer ohne einen weiteren Testvorgang immer wieder seine Duftkomposition zusammen mischen lassen kann. Das System läßt sich somit prägnant als Print, Smell und Mix beschreiben.

Das System, selbst wenn es nur zum Aufbringen einer Duftsubstanz ausgebildet ist, eignet sich hervorragend zum Aufbringen von Düften auf Briefpapier, Fotos, Etiketten und Verpackungen. Hierbei können die Duftsubstanz(en) und die Schrift getrennt oder bereits vorgemischt aufgetragen werden. Hier könnten z.B. mit Duftstoffen versehene Tintenkartuschen angeboten werden, die bei entsprechender Ansteuerung das Auftragen der Duftsubstanz gleichzeitig mit dem Aufbringen der Tinte ermöglichen. Duftsubstanzen und Tinte können auch in verschiedenen Behältern vorgesehen und wahlweise vor dem Auftragen vorgemischt werden. Hier können entweder nur die Duftsubstanzen oder auch die Duftsubstanzen und die Tinte vorgemischt werden.

Weiterhin könnten Produkte auf ihren Etiketten mit produktspezifischen Düften versehen werden, was insbesondere bei verpackten oder tiefgefrorenen Produkten interessant ist.

## Patentansprüche

1. Vorrichtung zum Generieren und Abfüllen von Duftkompositionen mit
- mehreren Behältern (38) zur Aufnahme unterschiedlicher Duftsubstanzen,
- wenigstens einer Auftrags- oder Austragsvorrichtung (34) zum Überführen einer Kleinstmenge von Duftsubstanzen aus den Behältern (38) auf die Haut, in die Luft oder auf einen Duftträger (12), z. B. Papier,
- mindestens einer Steuerung (32) für die Betätigung der Auftrags- bzw. Austragsvorrichtung (34),
- wenigstens einer Eingabeeinrichtung (24) für die Eingabe von Mengen-/Mischungsdaten der Duftsubstanzen in die Steuerung,
- wenigstens einem Rechner zum Generieren von Aktivierungs- und/oder Dosierungssignalen aus den Mengen-/Mischungsdaten
sowie mit
- wenigstens einer Abfüllstation (26) zum Abfüllen erzeugter Dultkompositionen in kleinen Mengen von 1 bis 500 ml, welche Station folgende Bestandteile umfaßt:
- mehrere Großbehälter (50) für die unterschiedlichen Duftsubstanzen,
- jeweils eine Mikroförderpumpe (52) für jede Duftsubstanz,
- eine zentrale Steuerung (48) für alle Mikroförderpumpen, und
- eine Eingabeeinrichtung (24) für den Erhalt der Mengen-/Mischungsdaten für die zentrale Steuerung (48),
- wobei die Ausgänge der Mikroförderpumpen in einen Befüllungsbereich für einen Kleinbehälter münden.

2. Vorrichtung nach Anspruch 1,
**dadurch gekennzeichnet, dass** die Steuerung (32) einen Speicher aufweist, in dem Identifikationsdaten betreffend die in den Behältern (38) enthaltenen Duftsubstanzen abgelegt sind, und dass ein Speicher mit einer Mischungsmatrix vorgesehen ist, in der die Mischbarkeit der Substanzen definiert ist.

3. Vorrichtung nach Anspruch 1 oder 2,
**dadurch gekennzeichnet, dass** die Auftrags-/Austragsvorrichtung(en) durch einen Druckkopf (34) in Piezo, Bubble-Jet oder Thermodrucktechnik gebildet ist/sind.

4. Vorrichtung nach einem der Ansprüche 1 bis 3,
**dadurch gekennzeichnet, dass** ein Druckkopf (34) zum Aufbringen einer Beschriftung und/oder maschinenlesbaren Kennung (18) auf den Duftträger (12) oder auf einen Bereich neben dem Duftträger vorgesehen ist.

5. Vorrichtung nach einem der vorhergehenden Ansprüche,
**dadurch gekennzeichnet, dass** die Abfüllstation (26) die Mischungs- und/oder Mengendaten über eine Datenleitung (28,30) oder einen maschinenlesbaren Datenträger (12,18) erhält.

6. Vorrichtung nach einem der vorhergehenden Ansprüche,
**dadurch gekennzeichnet, dass** die Eingabeeinrichtung der Abfüllstation einen Leser für eine maschinenlesbare Beschriftung aufweist.

7. Vorrichtung nach einem der vorhergehenden Ansprüche,
**dadurch gekennzeichnet, dass** der Kleinbehälter als Zwischenbehälter zum Mischen der Komposition der Duftsubstanzen ausgebildet ist.

8. Verfahren zum Aufbringen/Austragen einer Mischung von Duftsubstanzen auf einen Duftträger mittels einer Vorrichtung nach einem der Ansprüche 1-7, bei dem eine Duftmischung in einer Eingabevorrichtung ausgewählt wird, deren Mengen/Mischungsdaten durch einen Rechner in Aktivierungssignale für eine Auftrags/Austragsvorrichtung (34) umgerechnet werden, und eine Kleinstmenge der Duftmischung ausgetragen bzw. auf den Duftträger aufgebracht wird, wobei die Mengen-/Mischungsdaten direkt oder mittels maschinenlesbarer Datenträger an eine Abfüllstation (26) übertragen werden.

9. Verfahren nach Anspruch 8,
**dadurch gekennzeichnet, dass** mit der Aufbringung der Duftsubstanzen eine Beschriftung auf den Duftträger (12) oder einen Beschriftungsbereich neben dem Duftträger aufgebracht wird.

10. Verfahren nach Anspruch 8 oder 9,
**dadurch gekennzeichnet, dass** durch eine Steuerung die Mischbarkeit verschiedener Duftsubstanzen definiert bzw. unterbunden wird.

## Claims

1. A device for generating and dispensing fragrance compositions comprising
- several fonts (38) for accommodating a variety of fragrant substances,
- at least one applicator (34) for releasing a microsample of said fragrant substances from said fonts (38) to the skin, air or a scent carrier (12) e.g. paper,
- at least one controller (32) for actuating said applicator (34),
- at least one means (24) for entering scent-balancing data of said fragrant substances into said controller,
- at least one processor for generating release signals from said scent-balancing data
as well as
- at least one dispenser (26) for dispensing microsamples of said generated fragrance compositions from 1 to 500 m1, said dispenser comprising the following components:
- several reservoirs (50) for the various fragrant substances,
- one microdelivery pump (52) for each fragrant substance,
- a central controller (48) for all microdelivery pumps, and
- an entering means (24) for obtaining said scent-balancing data for said central controller (48),
- the outputs of said microdelivery pumps porting into a font filling zone.

2. The device as set forth in claim 1, **characterized in that** said controller (32) comprises a memory in which data identifying said fragrant substances contained in said fonts (38) are saved, and that a memory is provided with a balancing matrix defining the scent balance of said substances.

3. The device as set forth in claim 1 or 2, **characterized in that** said applicator(s) is/are formed by a print head (34) in a piezo, bubble jet or thermal printer.

4. The device as set forth in any of the claims 1 to 3, **characterized in that** a print head (34) is provided for applying print and/or a machine-readable code (18) to said scent carrier (12) or to a site alongside said scent carrier.

5. The device as set forth in any of the preceding claims, **characterized in that** said dispenser (26) receives said scent-balancing data via a data line (28, 30) or a machine-readable data carrier (12, 18).

6. The device as set forth in any of the preceding claims, **characterized in that** said entry means of said dispenser comprises a reader for machine-readable print.

7. The device as set forth in any of the preceding claims, **characterized in that** said font is configured as a buffer container for balancing the composition of said fragrant substances.

8. A method for applying a composition of fragrant substances to a scent carrier by means of a device as set forth in any of the claims 1 to 7 comprising the steps: selecting a fragrancy in an entry means whose scent-balancing data are converted by a processor into signals for activating an applicator (34) and releasing a microsample of said fragrancy to said scent carrier, said scent-balancing data being directly communicated or by means of a machine-readable data carrier to a dispenser (26).

9. The method as set forth in claim 8, **characterized in that** dispensing said fragrant substances is accompanied by application of print to the scent carrier (12) or to a print location alongside said scent carrier.

10. The method as set forth in claim 8 or 9, **characterized in that** a controller enables or disables balancing of various fragrant substances.

## Revendications

1. Dispositif pour générer et dispenser des parfums avec :
- plusieurs récipients (38) pour réceptionner différents parfums,
- au moins un dispositif d'application ou de répartition (34) pour transférer une toute petite quantité de parfums des récipients (38) sur la peau, à l'air ou sur un support de parfum (12), par exemple, du papier,
- au moins une commande (32) pour actionner le dispositif d'application ou de répartition (34),
- au moins un dispositif d'introduction (24) pour introduire des données quantitatives / de mélange sur les parfums dans la commande,
- au moins un calculateur pour générer des signaux d'activation et / ou des signaux de dosage à partir des données quantitatives / de mélange,
- au moins un poste de remplissage (26) pour dispenser les parfums produits en petites quantités allant de 1 jusqu'à 500 ml ; lequel poste comprend les éléments suivants :
- plusieurs gros récipients (50) pour les différents parfums,
- à chaque fois, une micro - pompe d'alimentation (52) pour chaque parfum,
- une commande centrale (48) pour toutes les micro - pompes d'alimentation, et
- un dispositif d'introduction (24) pour obtenir des données quantitatives / de mélange pour la commande centrale (48),
- les sorties des micro - pompes d'alimentation débouchant dans une zone de remplissage pour un petit récipient.

2. Dispositif selon la revendication 1, **caractérisé en ce que** la commande (32) comporte une mémoire dans laquelle sont stockées des données d'identification correspondant à celles des parfums stockés dans les récipients (38) et **en ce qu'**il est prévu une mémoire dotée d'une matrice de mélange dans laquelle la possibilité de mélange des substances est définie .

3. Dispositif selon la revendication 1 ou 2, **caractérisé en ce que** le (les) dispositif (s) d'application /de répartition est / sont formé / és par un bouton poussoir (34) selon une technique piézo, à jet de bulles ou à impression thermique.

4. Dispositif selon une des revendications 1 à 3, **caractérisé en ce qu'**un bouton poussoir (34) est prévu pour appliquer une inscription et / ou un marquage lisible par ordinateur (18) sur le support des parfums (12) ou dans la zone situé près du support de parfums.

5. Dispositif selon une des revendications précédentes, **caractérisé en ce que** le poste de remplissage (26) obtient les données quantitatives / de mélange par une ligne de données (28, 30) ou par un support de données (12, 18) lisible par un ordinateur .

6. Dispositif selon une des revendications précédentes, **caractérisé en ce que** le dispositif d'introduction du poste de remplissage comporte un lecteur pour une inscription lisible par ordinateur.

7. Dispositif selon une des revendications précédentes, **caractérisé en ce que** le petit récipient est formé pour servir de récipient intermédiaire pour mélanger les composants des parfums.

8. Procédé d'application / répartition d'un mélange de parfums sur un support de parfums au moyen d'un dispositif conforme à l'une des revendications 1 à 7 , dans lequel on choisit de mélanger les parfums dans un dispositif d'introduction dont les données quantitatives / de mélange seront transformées par un calculateur en signaux d'activation pour un dispositif d'application / répartition (34) et en ce qu'une petite quantité du mélange de parfums est répartie ou appliquée sur le support de parfums ; les données quantitatives / de mélange étant transférées directement ou au moyen d'un support de données lisible par un ordinateur à un poste de remplissage (26)

9. Procédé selon la revendication 8, **caractérisé en ce qu'**une inscription est appliquée sur le support de parfums (12) ou une zone d'inscription près du support de parfums en même temps que l'application des parfums .

10. Procédé selon la revendication 8 ou 9, **caractérisé en ce que** le mélange de différents parfums est défini, respectivement, ou interrompu par une commande.
